Europäisches Patentamt

**European Patent Office**

**Office européen des brevets**

(11) Numéro de publication: **0 184 511 B1**

(12) **FASCICULE DE BREVET EUROPEEN**

(45) Date de publication de fascicule du brevet: **14.04.93**

(51) Int. Cl.⁵: **G01N 33/531**, G01N 33/569, C12P 21/00, //(C12P21/00, C12R1:32)

(21) Numéro de dépôt: **85402376.9**

(22) Date de dépôt: **02.12.85**

Le dossier contient des informations techniques présentées postérieurement au dépôt de la demande et ne figurant pas dans le présent fascicule.

(54) **Procédé d'isolement de l'antigène mycobactériel A60 et utilisation de cet antigène pour la production de réactifs immunologiques et dans les méthodes d'essai immunologiques.**

(30) Priorité: **05.12.84 US 678470**

(43) Date de publication de la demande: **11.06.86 Bulletin 86/24**

(45) Mention de la délivrance du brevet: **14.04.93 Bulletin 93/15**

(84) Etats contractants désignés: **CH DE FR GB IT LI NL**

(56) Documents cités:
**US-A- 3 529 057**
**US-A- 4 123 427**

**BIOLOGICAL ABSTRACTS, vol. 71, no. 6, 1981, page 4021, colonne 2, résumé no. 38434, Philadelphia, PA, US; O. CLOSS et al.: "The antigens of Mycobacterium bovis, strain BCG, sudied by crossed immunoelectrophoresis: a reference system"**

(73) Titulaire: **ANDA BIOLOGICALS
37, rue de la Course
F-67000 Strasbourg(FR)**

(72) Inventeur: **Maes, Roland F.
10 A rue du 22 novembre
F-67000 Strasbourg(FR)**

(74) Mandataire: **Portal, Gérard et al
Cabinet Beau de Loménie 158, rue de l'Université
F-75340 Paris Cédex 07 (FR)**

Rank Xerox (UK) Business Services
(3.10/3.5x/3.0.1)

CHEMICAL ABSTRACTS, vol. 90, no. 21, 21 mai 1979, page 436, résumé no. 166472p, Columbus, Ohio, US; O. CLOSS et al.: "Antigenic analysis of Mycobacterium leprae"

CHEMICAL ABSTRACTS, vol. 87, no. 9, 29 août 1977, page 387, résumé no. 66433w, Columbus, Ohio, US; M. HARBOE et al.: "Production and assay of antibodies against one antigenic component of Mycobacterium bovis BCG"

INFECT. IMMUN. 1977, 16(2), 662-672;

SCAND. J. IMMUNOL. 1980, 12, 249-263;

## Description

La présente invention concerne essentiellement un procédé d'isolement de l'antigène mycobactériel A 60; un procédé de production de fragments immunologiquement actifs à partir de l'antigène A 60; l'utilisation de l'antigène A 60 ou de ses fragments immunologiquement actifs pour détecter dans un sérum d'un mammifère la présence d'anticorps contre l'antigène A 60; ou pour la production d'anticorps;un procédé de détection dans le sérum d'anticorps contre l'antigène A 60;et l'antigène A 60, ses fragments immunologiquement actifs en tant que produits nouveaux. Autrement dit, la présente invention se rapporte à l'antigène A 60 extrait de mycobactéries et à ses fragments immunologiquement actifs ainsi qu'à leur procédé d'isolement, l'utilisation en vue de la détection d'anticorps contre l'antigène A 60 afin de permettre par la suite l'établissement d'un diagnostic par le praticien, ainsi que la production d'anticorps spécifiques contre les antigènes ci-dessus.

On sait que les mycobactéries appartiennent à l'ordre des Actinomycétales. Ce sont des bactéries gram-positives faisant partie du supergroupe CMN, constitué par les Corynébactéries, les Mycobactéries et les Nocardia.

Les Mycobactéries se subdivisent en plusieurs groupes. Trois de ces groupes se différencient selon leur capacité à synthétiser des caroténoïdes. Ces trois groupes sont les photochromogènes (groupe I), les scotochromogènes (groupe II) et les non-chromogènes (groupe III). Un quatrième groupe consiste en des "bactéries à croissance rapide".

Les mycobactéries qui sont la cause de la lèpre chez les souris et les humains sont exclues de ces quatre groupes.

Un autre système de classification de ces bactéries se base sur leur pathogénicité, étant donné que certaines Mycobactéries appartenant à ces quatre groupes sont considérées pathogènes. Les représentants majeurs du groupe responsable de la tuberculose typique chez l'homme et les bovidés, dans le sens d'agents étiologiques typiques, sont M. tuberculosis et M. bovis. Les agents "atypiques", c'est-à-dire les pathogènes conditionnels de la tuberculose sont représentés par M. avium (pathogénique de façon stricte pour les oiseaux) , M. intracellulare, M. scrofulaceum, M. xenopi, M. ulcerans et M. kansasii. Ces agents, appartenant aux trois groupes mycobactériens majeurs, peuvent aussi provoquer de l'arthrite, des dermatites et des tuberculoses extrapulmonaires. La lèpre humaine est causée par M. leprae.

Les diagnoses conventionnelles de la lèpre et de la tuberculose sont basées sur des testscutanés utilisant des extraits bactériens inactivés; nommément de la léprosine et de la tuberculine. Ces extraits contiennent des impuretés qui peuvent provoquer des réactions allergiques.

Les tests cutanés les utilisant sont, de surcroît, de longue durée (1 à 3 jours) et imprécis dans la mesure où ils n'autorisent pas le suivi de l'évolution d'une maladie ou les effets d'une intervention chimiothérapique ou d'un traitement. La mise en évidence d'une réactivité altérée chez un patient au cours du temps n'est pas possible avec ces tests dont la réponse est simplement qualitative, se traduisant par "positif" ou "négatif".

Des testsdiagnostiques basés sur l'utilisation de sérum du malade et employant des réactifs spécifiques permettraient une surveillance épidémiologique plus pointue, une rapidité de diagnose très supérieure à celle offerte par les tests cutanés et éviteraient l'inconfort du patient.

Plus de 29 espèces de Mycobactéries sont recensées, certaines non-pathogènes, d'autres pathogènes conditionnels, d'autres induisant une tuberculose pulmonaire typique, et certaines provoquant d'autres maladies telles la lèpre, l'arthrite et la dermatite.

Ces bactéries sont des organismes complexes. M. tuberculosis était,à l'aube de la recherche dans ce domaine, supposé constitué de 11 antigènes majeurs; il est maintenant reconnu que le nombre des antigènes est beaucoup plus élevé. Certains de ces antigènes sont communs à plusieurs genres, c'est-à-dire qu'ils montrent une réactivité immunologique croisée entre des organismes appartenant au supergroupe Corynebactérium, Mycobactérium et Nocardia. D'autres antigènes sont communs à tous les membres du genre Mycobactérium et un troisième groupe d'antigènes est spécifique de l'espèce.

L'intérêt des chercheurs dans ce domaine s'est focalisé sur les antigènes mycobactériens spécifiques de l'espèce parce que leur utilisation permettrait la création d'un cadre taxonomique pour les Mycobactéries, ainsi que sur les antigènes dont la puissance antigénique était prometteuse pour l'élaboration de vaccins.

HARBOE et al. a décrit dans Infection and Immunity (1977) 16, N°2, pages 662-672, un procédé de production et d'essais d'anticorps contre un composant antigénique de Mycobacterium bovis BCG, à partir d'une préparation d'antigène 60 semi-purifiée par précipitation aux anticorps sur un gel d'agarose et chromatographie d'exclusion sur une colonne Séphadex G 200, par inoculation de lapin. Cependant, comme le reconnaît HARBOE lui-même, l'antigène 60 est présent avec d'autres antigènes (voir notamment la partie discussion aux pages 669 et 670).

En 1980, il était admis (Closs et al. (1980) : Scand. J. Immunol. 12, 249-263) que M. bovis -

(souche atténuée BCG) était composé d'un grand nombre d'antigènes révélés par immunoélectrophorèse bi-dimensionnelle. Ces antigènes sont tous identifiables et ont reçu un nombre antigènique caractéristique. A 60 présente un intérêt particulier parce qu'il est thermostable, un puissant immunogène et commun - sans être identique - à toutes les Mycobactéries analysées, s'étendant en fait à la totalité du supergroupe CMN. En particulier, l'A 60 de M. bovis et l'A 7 de M.leprae sont homologues (Harboe et al. (1979) Scand. J. Immunol. 9, 115-124).

La présente invention a donc pour but de résoudre le nouveau problème technique consistant en l'isolement de l'antigène A 60 sous une forme substantiellement pure, permettant de l'utiliser pour la détection de maladie dans des procédés de détection comprenant la formation de complexes antigène-anticorps.

Ce nouveau problème technique est résolu pour la première fois par la présente invention.

D'autre part, la présente invention a également pour but de fournir un procédé pour la production de fragments immunologiquement actifs à partir de l'antigène A 60 ainsi que leur utilisation dans un procédé de détection d'anticorps dans un sérum d'un mammifère malade.

La présente invention a également pour but de fournir un procédé de détection dans le sérum d'un mammifère, animal ou être humain, souffrant d'une maladie causée par une mycobactérie, de la présence éventuelle d'anticorps induits par ladite mycobactérie.

La présente invention permet donc de résoudre le problème technique précédemment mentionné et d'atteindre les buts mentionnés ci-dessus et ainsi que tout autre but qui sera apparent à l'homme du métier à partir de ce qui précède et de la description suivante.

En résumé, selon la présente invention, l'antigène A 60 a été isolé pour la première fois à partir de différentes Mycobactéries sous une forme substantiellement pure. L'antigène A 60 ainsi isolé a été utilisé pour détecter des maladies causées par diverses Mycobactéries, chez des mammifères, animaux ou êtres humains, ces maladies étant par exemple la tuberculose typique humaine et bovine, la tuberculose atypique, l'extra-tuberculose, la paratuberculose bovine,car il a pu être établi par le présent inventeur que les êtres malades contiennent des anticorps qui sont dirigés contre l'antigène A 60.

Ainsi, sur la base de cette découverte inattendue, l'antigène A 60 peut être utilisé pour détecter la présence de ces anticorps contre l'antigène A 60 dans les sérums de mammifères souffrant de ces maladies, en appliquant des procédés de détection immunologiques connus où de façon générale, l'antigène A 60 est mis en contact avec les sérums ou le plasma pour la formation de complexes avec des anticorps contre l'antigène A 60 contenu dans ces sérums ou plasma et le complexe est ensuite détecté de manière habituelle, par exemple en utilisant un procédé de traçage radiographique, ainsi qu'il est fait dans les techniques RIA, par marquage enzymatique, ainsi qu'il est fait dans les techniques ELISA, ou encore par l'agglutination de particules sensibilisées. On peut également utiliser l'antigène A 60 ainsi isolé pour la production d'anticorps polyclonaux et monoclonaux par injection de l'antigène A 60 dans des mammifères, spécialement des animaux, et récupération des anticorps ainsi produits. De surcroît, les antigènes A 60 peuvent être scindés par l'action de protéases connues et les fragments immunologiquement actifs obtenus par cette réaction de découpage peuvent êre récupérés et isolés.

Ainsi, selon un premier aspect, la présente invention fournit un procédé d'isolement de l'antigène mycobactériel A 60 à partir du cytoplasme d'une Mycobactérie appartenant au supergroupe CMN, caractérisé en ce qu'on réalise une destruction des parois cellulaires de ladite Mycobactérie ; on sépare les débris cellulaires, par exemple par centrifugation, et on récolte le surnageant contenant le cytoplasme; on réalise une chromatographie d'exclusion Sépharose 6B ou 4B ; et on récolte le pic d'exclusion contenant l'antigène mycobactériel A 60 substantiellement pur.

Selon une autre caractéristique du procédé d'isolement selon l'invention, on réalise l'isolement de l'antigène mycobactériel A 60 à partir d'une Mycobactérie photochromogène, en particulier M. kansasii ; d'une Mycobactérie scotochromogène, en particulier M. scrofulaceum ; à partir d'une Mycobactérie achromogène, en particulier M. xenopi ; à partir de la bactérie M. chelonei ; à partir de la bactérie M. paratuberculosis ou encore de la bactérie M.leprae ou M.bovis.

Selon une caractéristique préférée du procédé d'isolement selon l'invention, celui-ci est encore caractérisé en ce qu'on traite le surnageant avec du RNase ou DNase avant la chromatographie d'exclusion précitée.

Selon un deuxième aspect, la présente invention fournit aussi un procédé de production de fragments immunologiquement actifs à partir de l'antigène mycobactériel A 60, de préférence constitué par celui isolé par le procédé d'isolement ci-dessus mentionné, caractérisé en ce qu'on met en contact l'antigène mycobactériel A 60 avec une protéase ou un agent chimique ayant la fonction d'une protéase, tel que le bromure de cyanogène ; et on sépare les fragments immunologiquement actifs des autres fragments. De préférence, on réalise la séparation précitée par contact avec un agent adsorbant sensibilisé par un anticorps contre l'antigène mycobactériel A 60 de manière à former un complexe fragments immunologiquement actifs-anticorps anti-antigène mycobactériel A 60 que l'on récolte et on récupère las fragments immunologiquement actifs à partir de ce complexe.

Selon une caractéristique préférée de ce procédé, la récupération des fragments immunologiquement actifs est réalisée par chromatographie d'exclusion, de préférence sur colonne Séphadex G75, avantageusement à pH environ égal à 3 dans une solution molaire de (NH4) SCN.

Ce procédé peut également être appliqué à tous les antigènes mycobactériels A 60 obtenus à partir des bactéries précédemment mentionnées. On préfère l'appliquer spécialement aux antigènes mycobactérials A 60 obtenus à partir des Mycobactéries M.bovis ou de la Mycobactérie M.kansasii.

Selon encore un autre aspect, la présente invention concerne aussi l'utilisation de l'antigène mycobactériel A 60, dépourvu d'antigène contaminant, isolé de Mycobactéries du supergroupe CMN, ou de ses fragments immunologiquement actifs, pour détecter dans un sérum d'un mammifère la présence d'anticorps contre l'antigène mycobactériel A 60, en particulier dans un essai par réaction immunologique, par réaction d'agglutination, par réaction à la bentonite, par la réaction selon Ouchterlony, par électrophorèse en fuseau ou autre bien connu à l'homme de l'art.

Selon une variante de réalisation, on réalise la détection par un procédé RIA, ELA ou ELISA.

Cette utilisation concerne naturellement tous les antigènes mycobactériels A 60 pouvant être obtenus à partir des Mycobactéries du supergroupe CMN.

La présente invention concerne encore selon un autre de ses aspects, l'utilisation d'antigène mycobactériel A 60, dépourvu d'antigène contaminant, isolé de mycobactéries du supergroupe CMN, obtenu par le procédé précité ; ou de ses fragments immunologiquement actifs, pour la production d'anticorps en grande quantité par inoculation, ou vaccination, d'un mammifère, de préférence un animal, en particulier le lapin ou des poules, d'une quantité active d'antigènes mycobactériels A 60 ou de fragments immunologiquement actifs de celui-ci, suivi du développement par ledit mammifère inoculé d'anticorps contre ses antigènes aux fragments immunologiquement actifs, avec récupération de sérum de ce mammifère contenant les anticorps spécifiques. Dans le cas des poules, ceci est réalisé à partir du jaune d'oeuf produit par les poules.

Pour la détection dans le cadre du procédé type RLA, on utilise le procédé par marquage par un radio-isotope, de préférence $I^{125}$.

Enfin, la présente invention concerne également l'antigène mycobactériel A 60 en soi sous une forme substantiellement pure, dépourvue d'antigène contaminant, obtenu par le procédé d'isolement précité, ainsi que ses fragments immunologiquement actifs obtenus par le procédé de production précité.

Selon une variante de réalisation de l'invention, l'antigène mycobactériel A 60 est composé d'environ 57 % de protéines et d'environ 43 % de polysaccharides, et est obtenu à partir de la mycobactérie M.bovis, et de même pour ses fragments immunologiquement actifs.

Selon une autre variante de réalisation, l'antigène mycobactériel A 60 est composé d'environ 36,7 % de protéines et d'environ 63,3 % de polysaccharides et est obtenu à partir de la mycobactérie M.paratuberculosis, et de même pour ses fragments immunologiquement actifs.

L'invention concerne encore l'antigène mycobactériel A 60 sous une forme substantiellement pure, dépourvue d'antigène contaminant, obtenu par le procédé d'isolement précité, pour son application en tant que substance thérapeutiquement active, en particulier comme vaccin, en particulier contre la tuberculose ou la lèpre.

D'autres buts, caractéristiques et avantages de l'invention apparaîtront clairement à la lumière de la description explicative qui va suivre faite en référence aux dessins annexés dans lesquels :

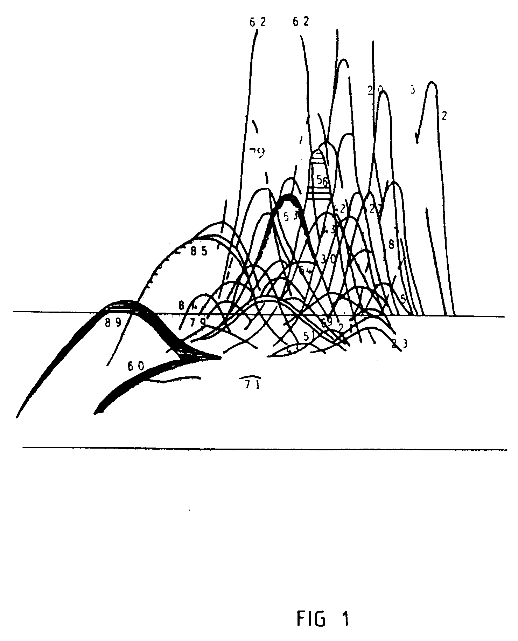

- la figure 1 illustre le système de référence pour les antigènes extra-cellulairas de M. bovis BCG obtenus par immunoélectrophorèse bi-dimensionnelle et colorés par le bleu de Coomassie, la seconde dimension étant obtenue par passage à travers un gel contenant de l'anti-sérum anti-BCG; et

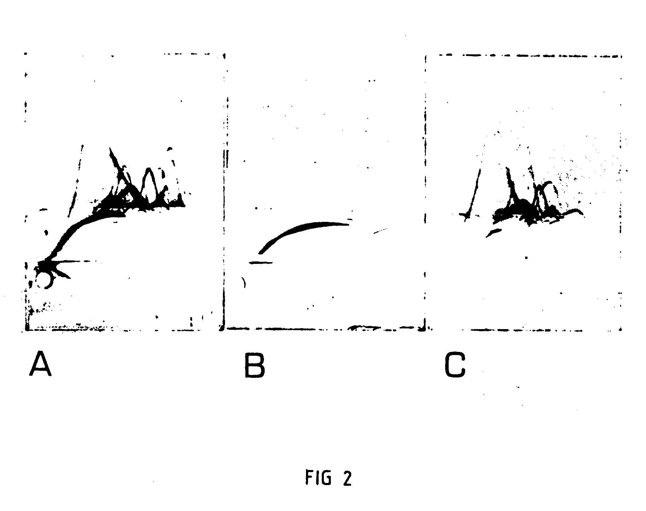

- la figure 2 montre le résultat d'une chromatographie d'exclusion réalisée selon l'invention, d'un échantillon cytoplasmique de M.bovis BCG où A est le cytoplasme de départ, B le pic d'exclusion constitué substantiellement seulement de l'antigène mycobactériel A 60 et C la pic d'inclusion contenant toutes les autres protéines.

Les Mycobactéries (excepté M.leprae et M. paratuperculosis) se multiplient dans le milieu de Dubos additionnés de 5% de sérum de cheval décomplémenté par chaffage à 56°C et durant 30mn. Elles sont récoltées à la fin de la phase de croissance par centrifugation et les parois bactériennes sont détruites par compression dans une cellule de pression (Aminco Instruments, Silver Spring, Md. USA) à environ 55,16 MPa (8.000 p.s.i.) et 4°C. L'antigène mycobactériel A 60 de diverses mycobactéries est facilement isolé et purifié par un procédé en une étape. Las homogénats des diverses Mycobactéries sont centrifugés à 2.000 r.p.m. durant 10 mn à 4°C pour écarter les débris cellulaires et le surnageant contenant le cytoplasme est récolté.

Par exemple, lorsqu'un échantillon cytoplasmique de M.bovis est soumis à une immuno-électrophorèse bidimensionnelle, les divers constituants antigéniques sont fractionnés selon un canevas illustré à la figure 1.

Spécifiquement, le canevas de référence de la figure 1 est obtenu en soumettant un échantillon, soit environ 24 μl d'échantillon cytoplasmique de départ dilué dans 10 μl de tampon barbital, à une électrophorèse en deux dimensions ou directions, la seconde direction de l'électrophorèse se réalisant à travers un gal d'agarose contenant de l'antisérum anti-BCG obtenu commercialement de Dako (Danemark), et ensuite coloration par le bleu de Coomassie.

La figure 2 montre les résultats obtenus avec le procédé d'isolement selon l'invention selon lequel le surnageant contenant le cytoplasme est soumis à une chromatographie d'exclusion en le faisant passer sur une colonne de gel d'exclusion et en récoltant le pic d'exclusion contenant l'antigène A 60 substantiellement pur (ici le pic B). Ainsi, on fait par exemple passer un échantillon cytoplasmique de M.bovis BCG consistant en 16,5mg de protéine solubilisée dans 1 ml, de préférence sur une colonne de Sépharose 6B.

A la figure 2, l'échantillon de départ est illustré en A et a un canevas similaire à celui de la figure 1. Le pic d'exclusion est représenté en B et consiste subtantiellement en antigène A 60 et le pic d'inclusion C contient tout le reste protéïnique, c'est-à-dire tous les autres antigènes présents dans l'échantillon.

Si désiré, le pic B, au cas où la présence de contaminants serait tout de même détectée, peut être rechromatographié sur la même colonne pour l'obtention de l'antigène A 60 sous une forme immunochimique homogène.

La composition chimique de l'antigène A 60 ainsi purifié par chromatographie d'exclusion sur une colonne de Sépharose 6B conformément à l'invention, a été analysée. On a trouvé qu'il est composé de 57% de protéines et de 43% de polysaccharides.

Le même procédé a été appliqué à la Mycobactérie M-paratuberculosis et on a trouvé que l'antigène A 60 isolé à partir de celle-ci est composé de 36,7% de protéines et 63,3% de polysaccharides.

L'unité de référence pour l'antigène A 60 tuberculeux a été établie de la façon suivante : 1 unité d'antigène A 60 est la quantité de l'antigène purifié par électrophorèse et coloré au bleu de Coomassie dont l'absorbance en solution à 530nm est :

$$A_{530}^{1\,cm} = 0,1$$

Cette unité spectrophotométrique correspond à 1,5 μg de composant protéinique et à 1,23 μg de composant polysaccharique. Elle correspond donc à 2,75 μg de l'antigène A 60 cytoplasmique.

L'antigène A 60 de M.bovis est le composant du cytoplasme bactérien migrant le plus lentement dans un champ électrophorétique (1% agarose dans le tampon Tris-barbital 0,02 M à pH 8,6); 8 volts/cm durant 1 heure à 15°C dans la première dimension et 3 volts/cm durant 18 heures à 15°C dans la deuxième dimension; détection des composants protéiniques par bleu de Coomassie).

La majorité de l'étude a été effectuée avec la souche atténuée Bacille Calmette-Guerin (BCG) de M. bovis.

Après avoir déterminé les conditions optimales pour l'isolement de l'antigène A 60, le même matériel, reconnu par sa location électrophorétique, a été isolé par la même technique selon l'invention de chromatographie d'exclusion à partir d'une mycobactérie photochromogène (groupe I, M.kansasii), d'une mycobactérie scotochromogène (groupe II, M.scrofulaceum), d'une mycobactérie achromogène (groupe III, M.xenopi) et d'une bactérie à croissance rapide (groupe IV, M.chelonei).

A ces représentants de divers groupes mycobactériens qui induisent un certain type de tuberculose (M.xenopi et M.scrofulaceum sont généralement non-pathogéniques) ont été ajoutés M.paratuberculosis qui infecte les bovidés et M.leprae qui a été obtenu par multiplication dans l'armadillo.

Selon le procédé d'isolement préféré selon l'invention, environ 15 mg de protéines libérées du cytoplasme de ces bactéries par la destruction des parois cellulaires de la Mycobactérie, sont introduites dans 1 ml de tampon (CaCl2 - 0,04M; Tris-HCl-0,02M; pH-7,4) et furent traitées durant 10 mn à 37°C par 10 unités de RNase, puis chromatographiés deux fois successivement sur une colonne à gel d'exclusion de Sépharose 6B et les antigènes A 60 respectifs de ces mycobactéries furent ainsi obtenus à l'état pur. Les échantillons appliqués à la colonne étaient 1 ml en volume; la colonne étant 10 ml en volume et le volume mort était de 3,5 ml.

Les protéines homologues d'antigène A 60 extraites de ces 7 Mycobactéries furent reconnues par leur comportement immuno-électrophorétique. Toutes les espèces mycobactériennes contenaient cette protéine en grande quantité. L'antigène A 60 de M.leprae fut reconnu comme étant l'antigène principal de la lépromine également dénommée A 7 dans un système de référence antérieur.

La production d'anticorps murins polyclonaux contre les antigènes A 60 isolés s'accomplit selon des procédures connues. Brièvement, des souris Balb/c furent sensibilisées par le pristane (0,5 ml I.P.). Cinq jours plus tard, 1 ml d'une suspension à 10% d'une solution aqueuse de l'antigène (10mg/ml) dans de l'adjuvant incomplet de Freund furent inoculés I.P. Quinze jours plus tard, on réalise deux inoculations de rappel effectuées chaque semaine. Les souris produisirent ainsi un ascite secrétant de grandes quantités d'anticorps. Les souris furent drainées tous les 3 jours et elles moururent en général après le 5ème ou 6ème drainage, de déshydratation.

Les anticorps monoclonaux furent obtenus dans les souris Balb/c selon des procédures connues.

Cinquante et un clones spécifiques pour l'antigène A 60 de M.bovis souche BCG furent isolés et 3 à 6 clones pour les 6 autres antigènes A 60 isolés.

L'obtention d'anticorps polyclonaux, d'anticorps monoclonaux à partir des antigènes A 60 substantiellement ou essentiellement purs précités, et d'une large quantité d'antigènes A 60 de grande pureté, isolés comme précédemment décrits, ont permis le développement du procédé de détection dans le sérum ou plasma de mammifères d'anticorps-antiantigène A 60 de manière à réaliser par la suite un diagnostic relativement à la présence ou l'absence de la maladie chez le mammifère auquel a été prélevé ledit sérum ou plasma.

Les exemples suivants permettent d'illustrer ce qui précède.

EXEMPLE I

Procédé général pour le marquage d'antigène A 60, son isolement à l'état marqué à l'état sensiblement pur et son utilisation dans un procédé de détection par essai RIA.

120 μg d'antigène A 60 isolé à l'état substantiellement pur comme précédemment décrit, sont mis dans 120 μl de tampon phosphaté salin à pH 7,2 (PBS) et incubés avec 3 μl de I$^{125}$ (300 uCi) et 10 μl de chloramine T (6,5 mg/ml dans le tampon phosphaté salin (PBS) pH 7,2) durant 3 mn à 20°C. La réaction est bloquée par addition de 10 μl de métabisulfite de sodium (8,45mg/ml dans un tampon phosphaté salin (PBS) à pH 7,2). Le volume final est porté à 200 μl avec PBS et chromatographié sur 2ml de Sépharose® 68. Le pic d'exclusion contenant l'antigène A 60 marqué à l'I$^{125}$ à l'état substantiellement pur est gardé en présence de 0,2% d'albumine et 0,02% de NaN$_3$.

On développe maintenant un essai radioimmunologique (essai RIA) pour la détermination de quantités minimes d'anticorps. De la protéine A de Staphylococcus aureus est adsorbée sur les parois de tubes en polystyrène. De l'antigène A 60 marqué à l'I$^{125}$ (environ 300 D.P.M. par μl) est ajouté à des quantités inconnues d'anticorps et le complexe obtenu est mis en contact avec la phase solide Proteine A. Après un temps d'incubation adéquat tout à fait apparent pour l'homme de l'art, on mesure la quantité de radioactivité attachée aux parois des tubes.

Cet essai radioimmunoiogique classique (essai RIA) est réalisé avec les différents anticorps obtenus et les 7 antigènes A 60 précédemment isolés qui sont marqués, ce qui a permis d'établir que tous les anticorps polyclonaux obtenus par inoculation de ces antigènes réagissaient largement (47% & 100%) avec les antigènes A 60 marqués introduits dans l'essai RIA. L'antigène A 60 paratuberculeux ne réagissait quant à lui que faiblement 27 à 30%).

Une étude approfondie a montré que les antigènes A 60 entraient en compétition de façon variable avec le dérivé protéinique purifié (P.P.D.) qui est une préparation purifiée de tuberculine (c'est-à-dire un extrait de BCG) et, de façon assez inattendue, avec la lépromine (un extrait de M.leprae) ainsi qu'avec les Corynebactériesdérivés-de lèpre (L.D.C.) pour la formation d'un complexe avec les anticorps polyclonaux testés. Ceci démontre que les antigènes A 60 de différentes espèces mycobactériennes, inclus les antigènes A 60 ne provoquant pas de tuberculose, contiennent au moins un épitope commun à toutes les espèces composant le supergroupe CMN. Une étude de la spécificité des différents anticorps monoclonaux à notre disposition a montré par ailleurs que la plupart d'entre eux interréagissaient à différents degrés.

Cependant, un anticorps absolument spécifique pour l'antigène A 60 isolé à partir de M.kansasii et un anticorps absolument spécifique également pour l'antigène A 60 isolé à partir de M.scrofulaceum furent isolés ainsi que 4 pour l'antigène A 60 isolé à partir de M.bovis. Aucun ne fut obtenu pour les autres espèces mycobactériennes analysées, sans doute parce que le nombre de clones secrétant isolés était trop faible.

EXEMPLE II.

Utilisation de l'antigène A 60 dans un essai enzymoimmunologique (Essai EIA).

L'antigène A 60 de M.bovis a été adsorbé aux parois de puits de plaques de microtitration en polystyrène (96 puits par plaque) pH 9,6 dons un tampon carbonate pH 9,6. Après adsorption, les puits furent remplis avec une solution 0,1% en albumine dans du tampon phosphaté salin à 4°C durant 18 heures puis les puits furent levés avec une solution 0,05% en Tween 20 dans du PBS et les puits furent finalement séchés et gardés secs à 4°C. L'antigène A 60 adsorbé sur les parois peut être gardé à 4°C au sec durant au moins 4 mois sans dégradetion observable de ses propriétés immunologiques.

Des anticorps de chèvre contre l'IgG humain furent purifiés par chromatographie d'affinité et marques à la peroxydase selon les techniques connues.

Un test enzymoimmunologique fut ainsi préparé où les anticorps anti-A 60 contenus dans un échantillon de sérum tel celui d'un tuberculeux, s'adborbent de façon spécifique sur les parois sensibilisées des puits de microtitration en se complexant avec l'antigène A 60 adsorbé. Après lavage avec du Tween® 20 à 0,05% dans du PBS, la présence d'anticorps spécifiques complexes est révélée par les anticorps anti-IgG couplés à de la peroxydase, comme indiqué ci-dessus.

Un test parallèle fut effectué où le même entigène A 60 (500 µg/ml) fut adsorbé à une suspension à 20% de latex (0,8 mµ de diamètre) dans un tampon glycine 0,1 m à pH 8,0). Après lavage des particules sensibilisées, une vérification de la sensibilisation fut réalisée par contact de 50 µl de particules avec 50 µl de dilution d'anticorps anti-A 60 sur une lame porte-objet. La limite de dilution de l'anticorps encore provoquant une agglutination était de 1 : 12 000. La compétition entre le latex sensibilisé à l'A 60 et de l'antigène A 60 libre pour cette limite de dilution agglutinante d'anticorps a montré que 2,6 µg d'A 60 libres étaient nécessaires pour inhiber l'agglutination induite par l'antisérum.

Ces deux tests qualificatifs ont été utilisés pour vérifier la présence d'anticorps anti-A 60 dans les sérums de malades reconnus tuberculeux par le test cutané usuel. Pour éviter les réactions non-spécifiques des sérums avec le latex, les sérums ont dû être dilués 6 fois dans du PBS avant leur introduction dans le test latex.

Une corrélation de 98% a été trouvée pour les réactions positives entre tests cutanés et l'ELISA décrit ci-dessus.

Des sérums prouvés négatifs par le test tuberculinique standard ont également été trouvés négatifs par cet ELISA, encore qu'on déceia 3% de faux positifs, sans doute parce que le test ELISA est plus sensible.

Le test rapide d'agglutination du latex est normalement moins sensible. Il ne décela que 94% des positifs mais tous les sérums négatifs dans le test tuberculin l'étaient également dans le test latex.

Ces résultats montrent que des anticorps anti-A 60 des Mycobactéries sont présents en grandes quantités dans le sérum des tuberculeux et que des tests diagnostiques - latex et ELISA -, basés sur la reconnaissance de ces anticorps dans le sérum sont des tests diagnostiques utiles.

EXEMPLE III.

Procédé de production de fragments immunologiquement actifs de l'antigène A 60.

Plusieurs protéases sont disponibles pour l'homme de l'art, en vue de scinder des protéines en fragments. Les enzymes les plus utilisés sont la trypsine, la papaïne, la chymotrypsine et la subtilysine encore que des enzymes plus ésotériques peuvent être utilisés ainsi que des produits chimiques tels le bromura de cyanogène. Ils scindent la protéine à la jonction de 2 acides aminés spécifiques et produisent, de ce fait, des fragments différents. Des expériences préliminaires sont nécessaires pour vérifier que la scission ne se produit pas à l'endroit où se localise un épitope immunologique important.

En nos mains, la trypsine s'est montrée adéquate et nous n'avons pas cherché l'effet d'autres enzymes.

L'antigène A 60 de M.bovis et de M.kansasii - (15mg dans 15ml) ont été traités par la trypsine selon un procédé connu. Brièvement, de la trypsine attachée à de la cellulose par l'intermédiaire de CNBr était incubée avec les solutions d'antigène A 60 dans un tampon constitué de solution de Hank à pH 7,2. Après 3 heures à 37°C, la trypsine attachée à la cellulose est éliminée par centrifugation.

La solution d'antigène A 60 M.bovis dégradée a été passée sur une colonne de cellulose - protéine A (Pharmacia, Uppsala; Suède) saturée avec l'anticorps Mab n° 31 obtenu à partir d'un clone isolé d'après la technique de Kohler - Milstein, à partir de lymphocytes de souris immunisées après une injection d'antigéne A 60 M.bovis, ledit anticorps possédant un large éventail d'activité immunologique pour l'antigène A 60. Après passage sur la colonne (1 x 4 cm), l'effluant fut récolté, puis le complexe antigène-anticorps fut détaché par passage d'une solution 3 M en (NH4) SCH à pH 3,0.

Le fragment immunologiquement actif d'antigène A 60 fut séparé de l'anticorps par chromatographie d'exclusion sur une colonne Séphadex® G 75 à pH 3,0 dans une solution molaire en (NH4) SCN.

Un procédé similaire fut appliqué pour les fragments d'antigèna A 60 de M.kansasii, passés sur protéine A Sepharose chargé en anticorps Mab n° 3 contre l'antigène A 60 M.kansasii, qui est rigoureusement spécifique pour cet antigène.

Le rendement en fragments immunologiquement actifs obtenus dans cette manipulation fut faible: environ 400 $\mu$g au total pour chaque fragment isolé. Ces fragments furent incorporés dans un adjuvant de Freund incomplet et la production d'anticorps essayée dans des souris (2 souris par échantillon, 50 $\mu$g de fragment I.D./dose/15 jours). Après 3 mois il a été trouvé que toutes les souris avaient des anticorps qui étaient monospécifiques pour l'A 60 M.kansasii dans un test d'immunodiffusion d'Ouchterlony, l'autre anticorps réagissant avec les 7 antigènes A 60 à notre disposition. Les anticorps anti-A 60 M.kansasii étaient moins abondants, dans les deux souris inoculées, démontrant que l'épitope qui avait induit cet anticorps spécifique était soit moins immunogénique ou/et que le fragment inoculé était plus court.

EXEMPLE IV.

Utilisation de l'antigène A 60 pour détecter des anticorps dans le sérum.

La facilité de développement d'un test EIA pour la sérodiagnose d'antigènes A 60 a permis une petite étude épidémiologique. L'antigène A 60 de 6 espèces mycobactériennes (M.kansasii, bovis, scrofulaceum, xenopi, chelonei et leprae) fut adsorbé à des plaques de microtitration (10 $\mu$g/ml dans du tampon carbonate à pH 9,6).

Les sérums des malades tuberculeux typiques, atypiques et extra-pulmonaires ont été analysés dons un test ELISA par contact avec ces antigènes (50 $\mu$l de sérum dilué 1 : 20 dans Tween® PBS). Les résultats de la recherche ont établi que tous les malades analysés ont montré la présence d'anticorps qui ont réagi de façon variable avec tous les antigènes A 60 utilisés. La variation, d'intensité de la coloration finale développée a mis en évidence une réactivité décrue de certains antigènes A 60 par rapport à d'autres. Le test a été ensuite amélioré par blocage des antigènes A 60 sur phase solide au moyen d'anticorps monoclonaux réagissant avec le déterminant universel CMN dominant (Mab 31 contre BCG). Une analyse de sérums de divers malades dans un test EIA prétraité par ce Mab 31 a donné un complément d'informations intéressant: 128 sérums analysés, dont 37 de tuberculose atypique, ont montré que M.xenopi, assumé apathogène. était l'agent causal de certaines (5 sur 37) tuberculoses atypiques. Aucun sérum réagissant violemment avec A 60 de M.scrofulaceum, leprae et chelanei n'a été détecté et seulement 9 sur 37 ont réagi avec M.kansasii. Le nombre d'observations et d'antigènes inclus dans l'analyse étaient trop réduits pour permettre d'autres conclusions. Quatre-vingt-dix-huit pourcent de sérums de tuberculeux typiques ont réagi avec l'antigène A 60 de M.bovis, malgré le bloquage du déterminant immunologique principal.

Ces exemples démontrent l'avantage qui peut être tiré de l'utilisation de l'antigène A 60 et de ses fragments pour des développements diagnostiques et thérapeutiques dans les domaines de la tuberculose et de la lèpre, mais ne peuvent bien entendu pas être restreints à ces seuls usages exposés. Il a par exemple été trouvé que l'élimination de composants carbohydratés de l'antigène améliorait la performance du test latex; également, on a trouvé que des quantités considérables d'anticorps spécifiques pouvaient être obtenues par extraction de gamma-globulines spécifiques de jaune d'oeuf de poules vaccinées. Egalement, l'antigène A 60 peut être isolé à partir du surnageant de culture bactérienne, à partir des ribosomes de bactéries ou à partir du cytosol. Il a également été trouvé que les ribosomes et le RNA ribosomal contaminent l'antigène A 60 préparé à partir de cytoplasme bactérien; un traitement de l'extrait avec du RNase et de la DNase avant la chromatographie d'exclusion améliorent considérablement la pureté du produit chromatographié. La Sepharose 4B ou tout autre gel d'exclusion peut être aussi efficace que la Sepharose 6B pour la séparation de l'antigène A 60 des autres constituants cytoplasmiques.

Les fragments immunologiquement actifs de l'A 60 peuvent être obtenus par d'autres enzymes que la trypsine. Enfin, d'autres tests diagnostiques que ceux ici employés, tels des tests d'agglutination, des tests à la bentonite, des tests d'Ouchterlony, des tests d'électrophorèse en fuseau et autres sont évidents pour l'homme de l'art.

On comprend ainsi que le procédé d'isolement selon la présente invention permet d'obtenir pour la première fois de l'antigène A 60 à l'état substantiellement pur, à partir du cytoplasme d'une mycobactérie appartenant au supergroupe CMN, ou d'une manière équivalente à partir d'une culture bactérienne, ou encore à partir des ribosomes de bactérie ou à partir du cytosol, par une chromatographie d'exclusion. Ce procédé est donc tout à fait général. De même, l'invention concerne le procédé général de production de fragments immunologiquement actifs des différents antigènes A 60 pouvant être obtenus à partir des différentes mycobactéries précitées.

L'invention couvre également l'utilisation de ces antigènes A 60 ou de leurs fragments immunologiquement actifs dans un procédé quelconque de détection d'anticorps dans un sérum ou plasma d'un mammifère, animal ou être humain en vue d'établir ultérieurement un diagnostic sur la présence ou l'absence de maladie.

L'invention concerne également tous las anticorps monoclonaux ou polyclonaux qui peuvent être obtenus à partir de tels antigènes A 60 ou de leurs fragments immunologiquement actifs car, de même que les clones, ils sont obtenus par des procédés de routine pour un homme du métier. Ainsi puisque les procédés décrits et revendiqués permettent d'aboutir obligatoirement et de manière reproductible, à l'antigène A 60 et à ses fragments immunologiquement actifs, un dépôt auprès d'un Institut agrée s'est avéré inutile. Il en est de même des clones ou anticorps que l'homme du métier obtiendra par de simples procédures de routine à partir de ceux-ci, et qui font aussi partie intégrante de l'invention.

## Revendications

1. Procédé d'isolement de l'antigène mycobactériel A 60 à partir du cytoplasme d'une mycobactérie appartenant au supergroupe CMN, caractérisé en ce qu'il comprend une étape de destruction des parois cellulaires de ladite mycobactérie; on sépare les débris cellulaires, notamment par centrifugation, et on récolte le surnageant contenant le cytoplasme; on réalise une chromatographie d'exclusion en faisant passer le surnageant contenant le cytoplasme sur une colonne de gel d'exclusion Sépharose® 6B ou 4B ; et on récolte le pic d'exclusion contenant l'antigène mycobactériel A 60 substantiellement pur.

2. Procédé d'isolement selon la revendication 1, caractérisé en ce que la mycobactérie utilisée est choisie parmi le groupe consistant d'une mycobactérie photochromogène, en particuler M.kansasii; d'une mycobactérie scotochromogène, en particuler M.scrofulaceum ; d'une bactérie achromogène, en particulier M.xenopi ; de M.chelonei ; de M.paratuberculosis, de M.leprae ou de M.bovis.

3. Procédé d'isolement selon l'une quelconque des revendications 1 à 2, caractérisé en ce que l'on traite le surnageant contenant le cytoplasme avec du RNase ou DNase avant la chromatographie d'exclusion.

4. Procédé de production de fragments immunologiquement actifs à partir de l'antigène mycobactériel A 60, isolé par le procédé selon l'une des revendications 1 à 3, caractérisé en ce qu'on met en contact l'antigène mycobactériel A 60 avec une protéase ou un agent chimique ayant la fonction d'une protéase, tel que le bromure de cyanogène, et on sépare les fragments immunologiquement actifs des autres fragments.

5. Procédé selon la revendication 4, caractérisé en ce qu'on réalise la séparation précitée par contact avec un agent adsorbant sensibilisé par un anticorps contre l'antigène mycobactériel A 60 de manière à former un complexe fragment immunologiquement actif-anticorps anti-antigène mycobactériel A 60 que l'on récolte ; et on récupère les fragments immunologiquement actifs à partir de ce complexe.

6. Procédé selon la revendication 5, caractérisé en ce qu'on effectue la récupération précitée en réalisant une chromatographie d'exclusion du complexe précité, de préférence sur colonne Séphadex® G 75 avantageusement à pH environ égal à 3 dans une solution molaire de-(NH$_4$)SCN.

7. Procédé selon l'une quelconque des revendications 4 à 6, caractérisé en ce qu'on produit des fragments immunologiquement actifs à partir de l'antigène mycobactériel A 60 isolé à partir des mycobactéries telles que définies à la revendication 2, en particulier à partir de M.bovis et de M.kansasii.

8. Antigène mycobactériel A 60, sous une forme substantiellement pure, dépourvu d'antigène contaminant, obtenu par le procédé d'isolement selon l'une quelconque des revendications 1 à 3.

9. Antigène mycobactéricl A 60 selon la revendication 8, composé d'environ 57 % de protéines et d'environ 43 % de polysaccharides, obtenu à partir de la mycobactérie M.bovis, et ses fragments immunologiquement actifs.

10. Antigène mycobactériel A 60 selon la revendication 8, composé d'environ 36,7 % de protéines et d'environ 63,3 % de polysaccharides, obtenu à partir de la mycobactérie M.paratubereulosis, et ses fragments immunologiquement actifs.

11. Fragments immunologiquement actifs de l'antigène mycobactériel A 60, obtenus par le procédé de production selon l'une quelconque des revendications 4 à 7.

12. Utilisation in vitro de l'antigène mycobactériel A 60, dépourvu d'antigène contaminant, isolé de mycobactéries du supergroupe CMN, obtenu par le procédé selon l'une quelconque des revendications 1 à 3 ; ou de fragments immunologiquement actifs de l'antigène mycobaetériel A 60, obtenus par le procédé de production selon l'une quelconque des revendications 4 à 7, pour détecter dans un sérum d'un mammifère la présence d'anticorps contre l'antigène mycobactériel A 60, en particulier par un essai par réaction immunologique, par un essai d'agglutination, par un essai à la bentonite, par un essai Ouchterlony ou par un essai d'électrophorèse.

13. Utilisation selon la revendication 12, caractérisé en ce qu'on détecte par un procédé RIA, EIA, ou ELISA.

14. Utilisation de l'antigène mycobactériel A 60, dépourvu d'antigène contaminant, isolé de mycobactéries du supergroupe CMN, obtenu par le procédé selon l'une quelconque des revendications 1 à 3 ou des fragments immunologiquement actifs de l'antigène mycobactériel A 60, obtenus par le procédé selon l'une quelconque des revendications 4 à 7, pour la production d'anticorps en grande quantité, par inoculation, ou vaccination d'un mammifère, en particulier un animal, d'une quantité active d'antigène mycobactériel A 60 ou de fragments immunologiquement actifs de celui-ci, suivi du développement par ledit mammifère inoculé d'anticorps contre ces antigènes ou fragments immunologiquement actifs, avec récupération de sérum de ce mammifère contenant les anticorps spécifiques.

15. Utilisation selon la revendication 14, caractérisée en ce que dans le cas des poules, les anticorps sont obtenus à partir du jaune d'oeuf produit par les poules.

16. Antigène mycobactériel A 60 sous une forme substantiellement pure, dépourvue d'antigène contaminant, obtenu par le procédé d'isolement selon l'une des revendications précédentes pour son application en tant que substance thérapeutiquement active, en particulier comme vaccin, notamment contre la tuberculose ou la lèpre.

**Claims**

1. Method for isolating the A60 antigen of Mycobacteria from the cytoplasm of a Mycobacterium belonging to the CMN supergroup, characterized in that it comprises a step consisting in destroying the cellular walls of said Mycobacterium; the cellular debris are separated, notably by centrifuging and the supernatant containing the cytoplasm is collected; an exclusion chromatography is performed by causing the supernatant containing the cytoplasm to pass on a Sepharose® 6B or 4B gel exclusion column; and the exclusion peak containing the substantially pure A60 antigen of Mycobacteria is collected.

2. Isolating method according to claim 1, characterized in that the Mycobacterium used is selected from the group consisting of a photochromogen Mycobacterium, in particular M. kansasii; of a scotochromogen Mycobacterium in particular, M.scrofulaceum; of an achromogen bacterium, in particular M.xenopi; of M.chelonei; of M.paratuberculosis, of M.Loprae or of M.bovis.

3. Isolating method according to any one of claims 1 to 2, characterized in that the supernatant containing the cytoplasm is treated with RNase or DNase before the exclusion chromatography.

4. Method for producing immunologically active fragments from the A60 antigen of Mycobacteria, isolated by the method according to one of claims 1 to 3, characterized in that the A60 antigen of Mycobacteria is placed in contact with a protease or a chemical agent having the function of a protease, such as cyanogen bromide, and the immunologically active fragments are separated from the other fragments.

5. Method according to claim 4, characterized in that said separation is effected by contact with an adsorbing agent sensibilized by an antibody against the A60 antigen of Mycobacteria so as to form a complex of immunologically active fragment and of an anti-A60 Mycobacterial antigen antibody which is collected; and the immunologically active fragments are recovered from this complex.

6. Method according to claim 5, characterized in that said recovery is achieved by carrying out an exclusion chromatography on said complex preferably on a G75 Sephadex® column advantageously at a pH which is approximately equal to 3 in a molar solution of $(NH_4)SCN$.

7. Method according to any one of claims 4 to 6, characterized in that immunologically active fragments are produced from the A60 antigen of Mycobacteria isolated from Mycobacteria such as defined in Claim 2, in particular from M.bovis and from M.kansasii.

8. A60 antigen of Mycobacteria, in substantially pure form, containing no contaminant antigen, obtained by the isolating method according to any one of claims 1 to 3.

9. A60 antigen of Mycobacteria according to claim 8, composed of about 57% protein and about 43% polysaccharides obtained from the M.bovis Mycobacterium, and its immunologically active fragments.

10. A60 antigen of Mycobacteria according to claim 8, composed of about 36.7% protein and about 63.3% polysaccharides, obtained from the M.paratuberculosis Mycobacterium, and its immunologically active fragments.

11. Immunologically active fragments of the A60 antigen of Mycobacterium, obtained by the production method according to any one of claims 4 to 7.

12. Use in vitro of the A60 antigen of Mycobacteria, containing no contaminant antigen, isolated from Mycobacteria of the CMN supergroup, obtained by the method according to any one of claims 1 to 3; or of immunologically active fragments of the A60 antigen of Mycobacteria, obtained by the production method according to any one of claims 4 to 7, for detecting in a mammal serum the presence of antibodies against the A60 antigen of Mycobacteria, in particular by an immunological reaction test, by an agglutination test, by a bentonite test, by an Ouchterlony test or by an electrophoresis test.

13. Use according to claim 12, characterized in that the detection is performed by a RIA, EIA, or ELISA method.

14. Use of the A60 antigen of Mycobacterium, containing no contaminant antigen, isolated from Mycobacteria of the CMN supergroups obtained by the method according to any one of claims 1 to 3 or immunologically active fragments of the A60 antigen of Mycobacteria, obtained by the method according to any one of claims 4 to 7, for the production of antibodies in large quantity, with inoculation or vaccination of a mammal, particularly an animal, of an active quantity of A60 antigen of Mycobacteria or with immunologically active fragments thereof, followed by the development by said inoculated mammal of antibodies against these antigens or immunologically active fragments, with recovery of serum from said mammal containing the specific antibodies.

15. Use according to claim 14, characterized in that in the case of hens, the antibodies are obtained from the egg yolk produced by the hens.

16. A60 antigen of Mycobacterium in substantially pure form, containing no contaminant antigen obtained by the isolating process according to one of the preceding claims, for its application as therapeutically active substance, in particular as a vaccine, notably against tuberculosis or leprosis.

**Patentansprüche**

1. Verfahren zur Isolierung des mycobakteriellen Antigens A 60 aus dem Cytoplasma eines der Obergruppe CMN angehörenden Mycobakteriums,
gekennzeichnet durch
Zerstörung der Zellwände des Mycobakteriums,
Abtrennung der Zellfragmente, insbesondere durch Zentrifugieren, und Gewinnung des das Cytoplasma enthaltenden Überstands,
Durchführung einer Chromatographie, indem man den das Cytoplasma enthaltenden Überstand über eine Säule mit dem Chromatographiegel Sepharose® 6B oder 4B laufen läßt, und
Gewinnung des Ausschlußpeaks, der das im wesentlichen reine mycobakterielle Antigen A 60 enthält.

2. Verfahren nach Anspruch 1, dadurch gekennzeichnet, daß das verwendete Mycobakterium ausgewählt wird unter den photochromogenen Mycobakterien, insbesondere M . kasasij, den scotochromogenen Mycobakterien, insbesondere M . scrofulaceum, den achromogenen Mycobakterien, insbesondere M . xenopi, M . chelonei, M . paratuberculosis, M . leprae und M . bovis.

3. Verfahren nach Anspruch 1 oder 2, dadurch gekennzeichnet, daß man den das Cytoplasma enthaltenden Überstand vor der Gelchromatographie mit RNase oder DNase behandelt.

4. Verfahren zur Herstellung von immunologisch aktiven Fragmenten aus dem mycobakteriellen Antigen A 60, das nach dem Verfahren nach einem der Ansprüche 1 bis 3 isoliert wurde, dadurch gekennzeichnet, daß man das mycobakterielle Antigen A 60 mit einer Protease oder einem chemischen Reagens mit der Funktion einer Protease, wie Bromcyan, in Kontakt bringt und die immunologisch aktiven Fragmente von den anderen Fragmenten trennt.

5. Verfahren nach Anspruch 4, dadurch gekennzeichnet, daß man die vorgenannte Trennung durch Inkontaktbringen mit einem Sorbens, das mit einem Antikörper gegen das mycobakterielle Antigen A 60 sensibilisiert wurde, so durchführt, daß sich ein Komplex aus immunologisch aktivem Fragment und einem gegen das mycobakterielle A 60-Antigen gerichteten Antikörper bildet, der gewonnen wird, und die immunologisch aktiven Fragmente aus diesem Komplex gewinnt.

6. Verfahren nach Anspruch 5, dadurch gekennzeichnet, daß die vorgenannte Gewinnung so durchgeführt wird, daß man eine Gelchromatographie des obengenannten Komplexes durchführt, vorzugsweise an einer mit Sephadex® G 75 gefüllten Säule, vorteilhaft bei einem pH-Wert von etwa gleich 3 in einer 1 M $NH_4SCN$-Lösung.

7. Verfahren nach einem der Ansprüche 4 bis 6, dadurch gekennzeichnet, daß man immunologisch aktive Fragmente aus dem mycobakteriellen Antigen A 60 herstellt, das aus Mycobakterien, wie in Anspruch 2 definiert, und insbesondere aus M . bovis und M . kansasij isoliert wurde.

8. Mycobakterielles Antigen A 60 in im wesentlichen reiner Form ohne kontaminierende Antigene, erhalten nach dem Verfahren nach einem der Ansprüche 1 bis 3.

9. Mycobakterielles Antigen A 60 nach Anspruch 8, das aus etwa 57 % Proteinen und etwa 43 % Polysacchariden besteht, erhalten aus dem Mycobakterium M . bovis, und seine immunologisch aktiven Fragmente.

10. Mycobakterielles Antigen A 60 nach Anspruch 8, das aus etwa 36,7 % Proteinen und etwa 63,3 % Polysacchariden besteht, erhalten aus dem Mycobakterium M . paratuberculosis, und seine immunologisch aktiven Fragmente.

11. Immunologisch aktive Fragmente des mycobakteriellen Antigens A 60, erhalten nach dem Verfahren nach einem der Ansprüche 4 bis 7.

12. In-vitro-Verwendung des mycobakteriellen Antigens A 60, das keine kontaminierenden Antigene enthält und aus Mycobakterien der Obergruppe CMN isoliert und nach dem Verfahren nach einem der Ansprüche 1 bis 3 erhalten wurde, oder von immunologisch aktiven Fragmenten des mycobakteriellen Antigens A 60, die nach dem Verfahren nach einem der Ansprüche 4 bis 7 erhalten wurden, zum Nachweis des Vorliegens von Antikörpern gegen das mycobakterielle Antigen A 60 im Serum von Säugetieren und Menschen, insbesondere durch immunologische Reaktion, Agglutinationstest, Bentonittest, Ouchterlony-Test oder elektrophoretischen Test.

13. Verwendung nach Anspruch 12, dadurch gekennzeichnet, daß zum Nachweis ein RIA-, EIA- oder ELISA-Text verwendet werden.

14. Verwendung des mycobakteriellen Antigens A 60, das keine kontaminierenden Antigene enthält und aus Mycobakterien der Obergruppe CMN isoliert und nach dem Verfahren nach einem der Ansprüche 1 bis 3 erhalten wurde, oder von immunologisch aktiven Fragmenten des mycobakteriellen Antigens A 60, die nach dem Verfahren nach einem der Ansprüche 4 bis 7 hergestellt wurden, zur Herstellung von Antikörpern in großer Menge durch Beimpfen oder durch Impfen eines Säugers, insbesondere eines Säugetiers, mit einer wirksamen Menge des mycobakteriellen Antigens A 60 oder von immunologisch aktiven Fragmenten davon, anschließende Entwicklung von Antikörpern gegen diese Antigene oder immunologisch aktiven Fragmente durch den geimpften Säuger und Gewinnung von die spezifischen Antikörper enthaltendem Serum von dem Säuger.

15. Verwendung nach Anspruch 14, dadurch gekennzeichnet, daß im Fall von Hühnern die Antikörper aus von den Hühnern produziertem Eigelb erhalten sind.

16. Mycobakterielles Antigen A 60 in im wesentlichen reiner Form, das keine kontaminierenden Antigene enthält und nach dem Verfahren nach einem der vorhergehenden Ansprüche erhalten wurde, zur Verwendung als therapeutischer Wirkstoff, insbesondere als Impfstoff, besonders gegen Tuberkulose oder Lepra.

FIG 1

FIG 2